# EUROPEAN PATENT APPLICATION

(11) **EP 4 613 241 A1**
(43) Date of publication of application: **10.09.2025**
(21) Application number: 25162202.3
(22) Date of filing: 06.03.2025
(51) Int. Cl.: A61F 2/06, A61B 17/12, A61F 2/24

(54) **IMPLANTABLE MEDICAL DEVICE INCLUDING VALVE MEMBER**

(30) Priority: 06.03.2024 US 202463561995 P
(71) Applicant: Cook Medical Technologies, LLC, Bloomington, IN 47404 (US)
(72) Inventor: EVANS, Douglas W., Indiana, 47906 (US); KRATZBERG, Jarin A., Indiana, 47906 (US); KÖLBEL, Tilo, 20251 Hamburg (DE)
(74) Representative: Mathys & Squire

(57) **Abstract**

An implantable medical device for preventing backflow into a false lumen of dissection includes an elongate support member, an elongate flexible tubular valve member having a length, a proximal end, and an open distal end. At least one elongate resilient member extends at least partially along the length of the elongate flexible tubular valve member, the at least one elongate resilient member having a proximal end distal to the elongate support member, a distal end proximal to the open distal end of the elongate flexible tubular valve member, an elongated configuration, and a coiled configuration. The at least one elongate resilient member is unattached to the elongate support member. The at least one elongate resilient member, upon release from a constraining force, is configured to move from the elongated configuration to the coiled configuration thereby closing the open distal end of the elongate flexible tubular valve member.

## Description

### CROSS-REFERENCE TO RELATED APPLICATIONS

This application claims the benefit of U.S. Provisional Patent Application No. 63/561,995, filed March 6, 2024, each of which are hereby incorporated herein by reference in their entirety.

### TECHNICAL FIELD

The present invention relates to an implantable medical device including an occluder, for example, an occluder for a false lumen of dissection.

### BACKGROUND

A false lumen caused by a type B aortic dissection may be treated by closing the upstream end of the false lumen, for example, with a stent graft. However, in some instances, the false lumen can still receive backflow from downstream tears in the false lumen wall. A conventional way to treat this is to use a candy plug, particularly a candy plug including a valve element.

Some existing documents include U.S. Pat. Nos. 9,364,354, 7,278,430, 9,427,233, 6,926,689, and 11,229,438, "Distal False Lumen Occlusion in an Aortic Dissection with a Homemade Extra Large Vascular Plug: The Candy-Plug Technique" by Kölbel et al., J Endovasc Ther. 2013 August; 20(4): 484-9, CN203852449, and WO 2008/097590. An implantable medical device configurated to occlude an aortic dissection is disclosed in U.S. Patent No. 11,229,438, the contents of which are hereby incorporated by reference in their entirety. Another implantable medical device is disclosed in U.S. Patent Pub. No. 2022/0395278, the contents of which are hereby incorporated by reference in their entirety. A closure mechanism is disclosed in U.S. Patent Pub. No. 2022/0265416, the contents of which are hereby incorporated by reference in their entirety.

### SUMMARY OF THE INVENTION

The present invention seeks to provide an improved implantable medical device for preventing backflow into a false lumen of dissection.

An implantable medical device for preventing backflow into a false lumen of dissection is disclosed that includes an elongate support member having an internal wall forming an internal lumen, a proximal end, and a distal end, and an elongate flexible tubular valve member having a length, an open proximal end, and an open distal end. The proximal end of the elongate flexible tubular valve member extends from the elongate support member and is configured to be held open thereby in a deployed condition of the implantable medical device. The elongate flexible tubular valve member is unstented along its length. The implantable medical device also includes at least one elongate resilient member extending at least partially along the length of the elongate flexible tubular valve member. The at least one elongate resilient member has a proximal end distal to the elongate support member, a distal end proximal to the open distal end of the elongate flexible tubular valve member, an elongated configuration, and a coiled configuration. The at least one elongate resilient member is unattached to the elongate support member. The at least one elongate resilient member, upon release from a constraining force, is configured to move from the elongated configuration to the coiled configuration thereby closing the open distal end of the elongate flexible tubular valve member.

Further, the at least one elongate resilient member may be interwoven with the elongate flexible tubular valve member at least partially along the length of the elongate flexible tubular valve member. Each of the proximal end and the distal end of the at least one elongate resilient member may include a retention structure configured to prevent removal of the at least one elongate resilient member from the elongate flexible tubular valve member. The retention structure may be a coil. Additionally, the at least one elongate resilient member may also be at least one shape-memory material member. The at least one shape-memory material member may be at least one pre-coiled nitinol wire. Also, the elongate flexible tubular valve member may taper from its proximal end to its distal end along at least part of its length. Further, the at least one elongate resilient member may be two elongate resilient members. The two elongate resilient members may be located diametrically opposite partially along the length of the elongate flexible tubular valve member.

There is also provided an implantable medical device for preventing backflow into a false lumen of dissection that includes an elongate support member having an internal wall forming an internal lumen, a proximal end, and a distal end, and a flexible valve member extending distally of the elongate support member. The flexible valve member includes a length, an open proximal end, and an open distal end. The proximal end of the flexible valve member is configured to be held open by the elongate support member in a deployed condition of the implantable medical device. The implantable medical device also includes at least one resilient member extending at least partially along the length of the flexible valve member. The at least one resilient member has a proximal end and a distal end that each terminate distally of and are unattached to the elongate support member. The at least one resilient member is also configured to close the open distal end of the flexible valve member in the deployed condition of the implantable medical device.

Further, the at least one resilient member may be interwoven with the flexible valve member at least partially along the length of the flexible valve member. At least one of the proximal end and the distal end of the at least one resilient member may be configured to prevent removal of the at least one resilient member from the flexible valve member. At least one of the proximal end and the distal end of the at least one resilient member may include a retention structure configured to prevent such removal. The retention structure may be a coil. Additionally, the at least one resilient member may be two resilient members, and the two resilient members may be located diametrically opposite partially along the length of the flexible valve member. Also, the at least one resilient member may be at least one shape-memory material member. The at least one shape-memory material member may be at least one pre-coiled nitinol wire.

Further, the at least one resilient member may be configured to fold and/or coil close the open distal end of the flexible valve in the deployed condition of the implantable medical device. Additionally, the at least one resilient member may include an elongated configuration and a coiled configuration where, upon release from a constraining force, the at least one resilient member is configured to move from the elongated configuration to the coiled configuration thereby closing the open distal end of the elongate flexible tubular valve member. Also, the elongate support member may include at least one stent where the at least one resilient member is unattached to the at least one stent.

There is also is provided an implantable medical device for preventing backflow into a false lumen of dissection that includes a graft material having an internal wall forming an internal lumen. The graft material extends from a proximal end of an elongate support member to an open distal end of a flexible valve member. The flexible valve member extends distally from the elongate support member and has an open proximal end configured to be held open by the elongate support member. The implantable medical device also includes at least one stent supporting the internal wall of the elongate support member and at least one resilient member extending partially along a length of the flexible valve member and being unattached to the at least one stent. The flexible valve member is unstented. The at least one resilient member includes a tendency to coil and/or fold close the open distal end of the flexible valve member.

Further, the at least one resilient member may be interwoven with the graft material. Additionally, the at least one resilient member may terminate distally to the elongate support member and proximally to the open distal end of the flexible valve member. At least one of a proximal end and a distal end of the at least one resilient member may be atraumatic. Also, at least one of a proximal end and a distal end of the at least one resilient member may include a retention structure configured to prevent removal of the at least one resilient member from the flexible valve member. The retention structure may be a coil.

Further, the at least one resilient member may be at least one shape-memory material member. The at least one shape-memory material member may be at least one pre-coiled nitinol wire. Additionally, the flexible valve member may be an elongate flexible tubular valve member. The elongate flexible tubular valve member may taper from its proximal end to its open distal end along at least part of its length. The at least one resilient member may be two elongate resilient members where the two elongate resilient members are located diametrically opposite partially along the length of the elongate flexible tubular valve member. Also, the at least one resilient member may include an elongated configuration and a coiled configuration where, upon release from a constraining force, the at least one resilient member is configured to move from the elongated configuration to the coiled configuration thereby closing the open distal end of the flexible valve member.

### BRIEF DESCRIPTION OF THE DRAWINGS

Embodiments of the invention are described below, by way of example only, with reference to the accompanying drawings, in which:
FIG. **1** is a schematic representation of a type B aortic dissection with a false lumen closed at its upstream end;
FIG. **2** is a schematic representation of a type B aortic dissection showing how back flow from downstream tears can be plugged using a known candy plug;
FIG. **3** is a front view of an implantable medical device according to an embodiment of the invention in an elongated configuration and configured to occlude a false lumen of an aortic dissection;
FIG. **4** **is** a view of at least one resilient member of the implantable medical device of FIG. **3** in the elongated configuration;
FIG. **5** is a partial front view of an elongate flexible tubular valve member of the implantable medical device of FIGS. **3-4** in the elongated configuration;
FIG. **6** is a partial side view of the elongate flexible tubular valve member of the implantable medical device of FIGS. **3-5** in the elongated configuration;
FIG. **7** is a front view of the implantable device of FIGS. **3-6** in the elongated configuration condition and disposed over an inner cannula of a delivery device;
FIG. **8** is a perspective view of the implantable medical device of FIGS. **3-7** in the elongated configuration;
FIG. **9** is a side view of the implantable medical device of FIGS. **3-8** in the elongated configuration;
FIG. **10** is an end view of the implantable medical device of FIGS. **3-9** in the elongated configuration;
FIG. **11** is a partial front view of the elongate flexible tubular valve member of the implantable medical device of FIGS. **3-10** in the elongated configuration;
FIG. **12** is a partial side view of the elongate flexible tubular valve member of the implantable medical device of FIGS. **3-11** in the elongated configuration;
FIG. **13** is an end view of the implantable medical device of FIGS. **3-12** in a coiled configuration;
FIG. **14** is an end view of the implantable medical device of FIGS. **3-13** in the coiled configuration; and
FIG. **15** is a side view of the implantable medical device of FIGS. **3-14** in the coiled configuration.

### DETAILED DESCRIPTION

Described below are preferred embodiments of an implantable medical device constructed according to the teachings herein. It is to be understood that the drawings are not to scale and are intended to be merely illustrative of the features and elements of the device and its components.

Throughout this specification, the term proximal with respect to both human or animal vasculature will be used to refer to the region closest to the heart and, similarly, the part of the implantable medical device that is closest to the heart when in use. The term distal will be used for the regions of the human or animal vasculature further from the heart and, similarly, those parts of the implantable medical device that are further from the heart when in use. With regard to a deployment or introducer assembly or retrieval device, the term distal is also used to denote the part of the assembly that remains closest to the clinician during the medical procedure and typically outside the patient. The term proximal is also used to denote the end of the assembly that is furthest from the clinician, which is first fed endoluminally into the patient's vasculature. Proximal may also be used to designate an inflow end of an endoluminal prosthesis and distal may also be used to designate an outflow end of an endoluminal prosthesis.

A proximal direction is a direction that would cause an object to assume a more proximal position. A distal direction is a direction that would cause an object to assume a more distal position.

The implantable devices described herein have what could be termed a compressed configuration or a compressed condition for delivery and a deployed configuration or a deployed condition upon implantation. Unless otherwise specified, the description of the devices relates to the deployed condition.

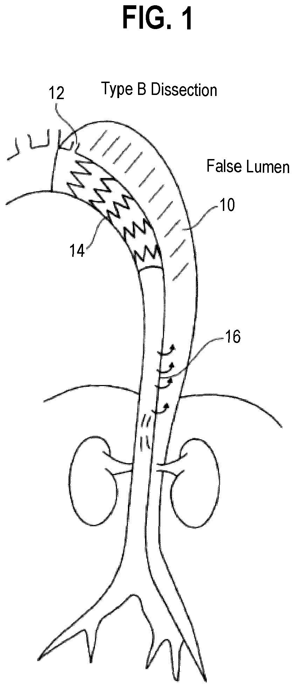

FIG. **1** shows a representation of a type B aortic dissection which has resulted in a false lumen **10.** An upstream tear **12** in the aortic wall has been closed by a stent graft **14.** However, the stent graft **14** does not extend all the way to the downstream end of the false lumen. In this case, there is a downstream tear **16** in the false lumen wall which allows backflow into the false lumen. This can prevent the false lumen from draining and put additional strain on the dissection.

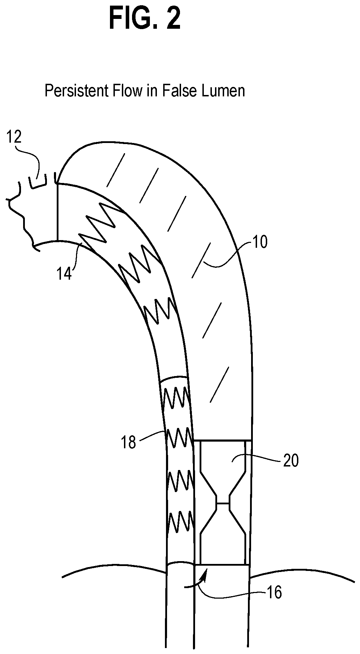

FIG. **2** is a representation of a known way of addressing the problem shown in FIG. **1****.** As can be seen in FIG. **2****,** a further stent graft or stent **18** has been deployed downstream of the first stent graft **14** in the aorta. Suitable stent grafts and stents for treating dissection tears and their method of construction are disclosed in U.S. Publication No. 2006/0142836, the contents of which are incorporated by reference in their entirety. In addition, a candy plug **20** has been deployed in the false lumen.

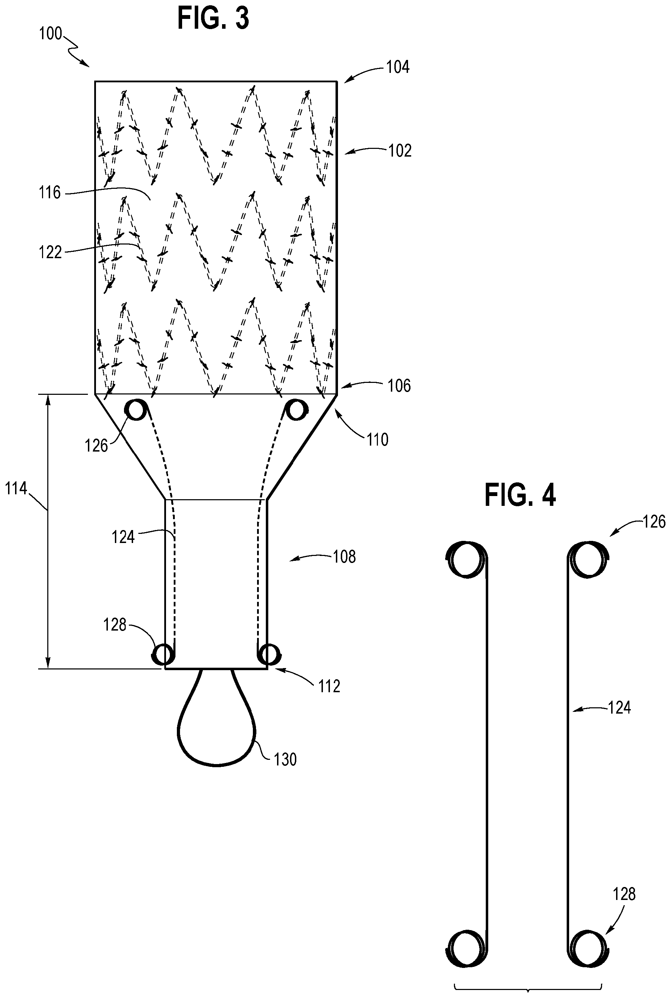

FIGS. **3-15** depict an implantable medical device **100** for preventing backflow into a false lumen of dissection. As can be seen in FIGS. **3** and **8****-9,** the device **100** includes an elongate support member **102** in the form of a stent graft and an elongate flexible tubular valve member **108** (shown in an elongated configuration). The support member **102** includes a proximal end **104** and a distal end **106** and is substantially cylindrically shaped. The valve member **108** includes a length **114,** a proximal end **110,** and an open distal end **112.** The support member **102** supports the valve member **108** to prevent backflow into the false lumen by occlusion of the lumen. The valve member **108** is substantially entirely unstented along its length **114.** The valve member **108** extends from the distal end **106** of the support member **102** and tapers from the distal end **106** of the support member **102** to a narrower configuration. Hence, the diameter of the support member is greater than the diameter of the valve member.

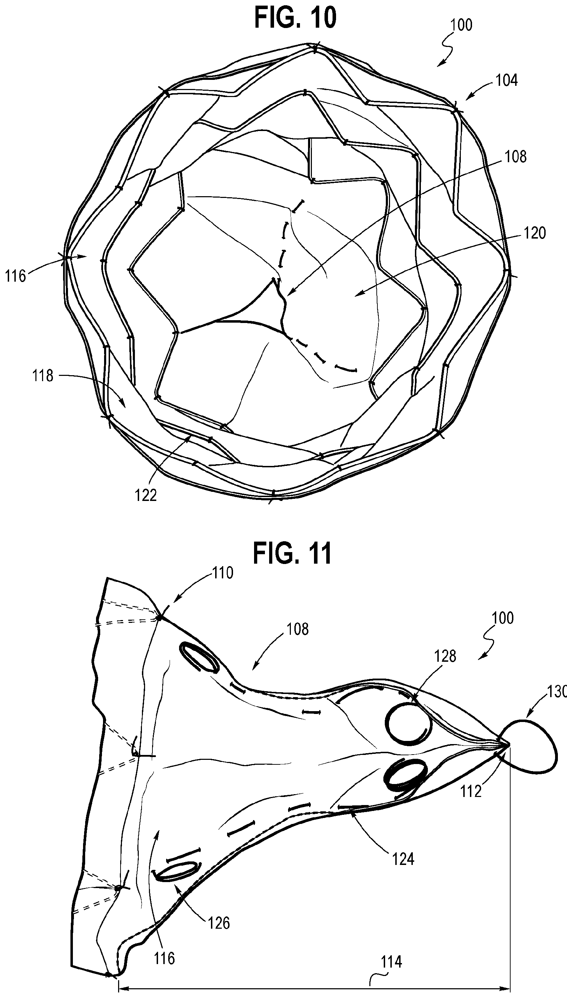

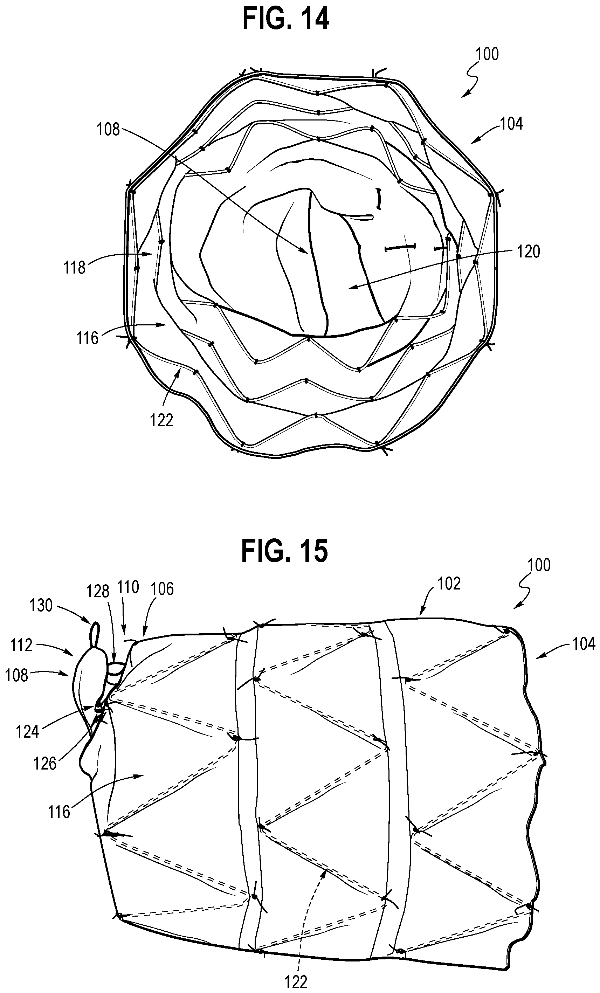

The support member **102** comprises a graft material **116** extending from the proximal end **104** to the distal end **106.** The graft material **116** provides an internal wall **118** forming an internal lumen **120** that is continuous through the support member **102** (as shown in FIGS. **10** and **14****).** While the term graft material is used throughout this specification, it is to be understood that graft material is not limited to only conventional graft material; it does not exclude that other flexible materials may be used, provided they are suitable for implantation in a human or animal body. The material of the support member **102** and valve member **108** is preferably substantially impermeable to blood. In addition, the internal wall **118** of the support member **102** is preferably substantially impermeable to blood and is preferably configured to seal against the walls of a false lumen in such a way as to prevent backflow from passing around the valve member **108.**

The support member **102** is provided with at least one stent **122** to support the internal wall **118** and hold it against vessel walls and to hold open the proximal end **110** of the valve member **108** in a deployed condition of the device **100.** The at least one stent **122** may be sewn to the graft material **116** at various points. One skilled in the art will appreciate that there are other means of securing or attaching the at least one stent **122** to the graft material **116,** for example, by using a suitable adhesive, encapsulation, and the like.

In the depicted embodiment, there are three internal stents **122:** a proximal stent, a distal stent, and an intermediate stent. In alternate embodiments, different numbers of stents **122** may be provided. The stents **122** may also have different arrangements regarding whether they are internal, external, or combinations thereof. Various ones of the at least one stent **122** may also be provided with gold markers or any radiopaque marker.

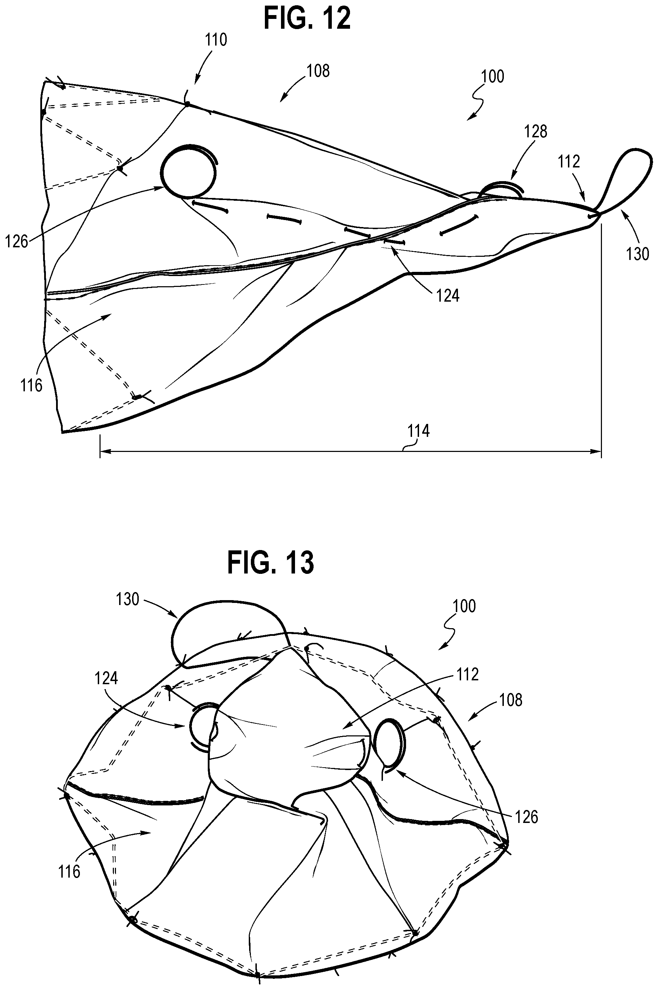

As set forth above, the support member **102** is substantially cylindrical, at least in the deployed condition (as seen in FIGS. **13-15****).** In the deployed condition, the support member **102** has a diameter of about 46 mm and a length of about 71 mm. Though, these dimensions may be varied to suit the treatment and/or patient. For example, the diameter may have a range of from about 20 mm to about 50 mm and the length may have a range of from about 40 mm to about 80 mm.

As shown in FIGS. **3** and **8****-9,** the valve member **108** extends distally of the distal end **106** of the support member **102.** The valve member **108** and the support member **102** may be constructed of a single piece of material. Alternatively, the proximal end **110** of the valve member **108** is secured and/or attached to the support member **102.** The distal end **106** of the support member **102** is configured to hold open the proximal end **110** of the valve member **108.** At least in the deployed condition of the device **100,** the diameter of the proximal end **110** of the valve member **108** is approximately the same as the diameter of the distal end **106** of the support member **102.**

In FIGS. **3****, 8-10,** and **14,** the graft material **116** extends and/or continues from the distal end **106** of the support member **102** to form the valve member **108.** In other words, the graft material **116** extends and/or is continuous from the proximal end **104** of the support member **102** to the distal end **112** of the valve member **108.** Accordingly, the internal wall **118** and internal lumen **120** extends and/or is continuous from the proximal end **104** of the support member **102** to the open distal end **112** of the valve member **108.** The valve member **108** may alternatively be referred to as a tail section of the device **100.**

Alternatively, the valve member **108** may not be formed from the continuous graft material **116** of the support member **102.** The proximal end **110** of the valve member **108** is secured to the support member **102** and configured to be held open thereby in the deployed condition of the device **100** to provide a proximal opening of the valve member **108.** The internal wall **118** of the support member **102** and an internal wall of the valve member **108** may be substantially continuous, and the internal lumen **120** of the support member **102** and an internal lumen of the valve member **108** are in fluid communication. The proximal end **110** of the valve member **108** may be secured to the internal wall **118** of the support member **102** around a circumference of the proximal end **110** of the valve member **108** around an inner circumference of the internal wall **118** of the support member **102** at an attachment support section. The proximal end **110** of the valve member **108** is secured to the support member **102** by being sewn to the support member **102.** However, a person skilled in the art will appreciate that other means of securing or attaching the proximal end **110** of the valve member **108** to the support member **102** are possible, for example using a suitable adhesive.

Additionally, in alternative embodiments, the valve member **108** may continue from or be attached to the proximal end **104** of the support member **102.** In such embodiments, the valve member **108** may extend proximally to the support member **102** or distally into the internal lumen **120** of the support member **102.** In embodiments where the valve member **108** extends into the internal lumen **120** of the support member **102,** the proximal end **110** of the valve member **108** may be connected to the proximal end **104** of the support member **102** and the distal end **112** of the valve member **108** may be located within the internal lumen **120** of the support member **102.** The valve member **108** of these embodiments may be considered to be inverted. As stated above, U.S. Patent No. 11,229,438 and its contents are incorporated by reference in their entirety, including FIGS. 14, 15, 17, and 18 and the relevant disclosure thereto.

Referring again to FIGS. **3** and **8****-9,** the valve member **108** may be elongated, flexible, tubular, and/or combinations thereof. The valve member **108** may also be substantially unstented. The length **114** of the valve member **108** is loose from the support member **102** and is compressible and foldable from its distal end **112** to its proximal end **110.** In the depicted embodiment, the valve member **108** is unstented along its length **114** and is an elongated flexible tubular valve member **108.** As noted above, the support member **102** supports the proximal end **110** of the valve member **108.** More specifically, the distal end **106** of the support member **102** supports the proximal end **110** of the valve member **108.** The proximal end **110** of the valve member **108** is open in the deployed condition of the device **100,** as shown in FIGS. **13-15****.**

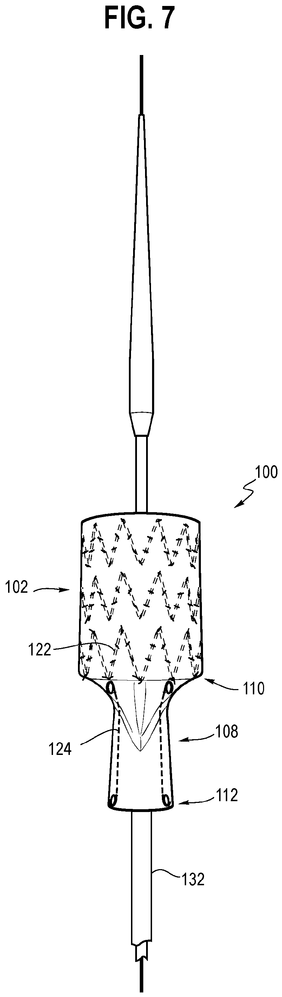

The open distal end **112** of the valve member **108** is closable upon release of the medical device **100** from a delivery system. For example, the distal end **112** of the valve member **108** is configured or designed to only be opened when disposed on its delivery system and/or to allow components of an introducer system to be retracted, and otherwise to be closed. As seen in FIGS. **7** and **10****,** the distal end **112** is open, for example, to allow retraction of an introducer tip, cannula, and/or wire-guide. However, as discussed in more detail below, at least one resilient member **124** causes the distal end **112** to be closed in the deployed condition to minimize back flow into a false lumen. This can assist blood in the false lumen to thrombose. The closed distal end **112** of the valve member **108** in the deployed condition is depicted in FIGS. **13-15****.**

As shown in FIGS. **3** and **8****-9,** and described above, the valve member **108** tapers from the proximal end **110** to the open distal end **112** along at least part of its length **114.** As a result, the diameter of the distal end **112** is smaller than the diameter of the proximal end **110** of the valve member **108.** The taper may be linear or non-linear, and in alternative embodiments, the valve member **108** may not include a taper. In this embodiment, the diameter of the valve member **108** tapers from about 46 mm at its proximal end **110** to about 12 mm at its distal end **112** and has a tolerance of about 4 mm. The length **114** of the valve member **108** is approximately 40 mm. Though, these dimensions may be varied to suit the treatment and/or patient. For example, the diameter of the valve member **108** at its proximal end **110** may have a range of from about 20 mm to about 50 mm; the diameter of the valve member **108** at its distal end **112** may have a range from about 12 mm to about 24 mm; the length **114** of the valve member **108** may have a range of from about 25 mm to about 60 mm. Accordingly, the length of the device **100** may have a range from about 65 mm to about 140 mm.

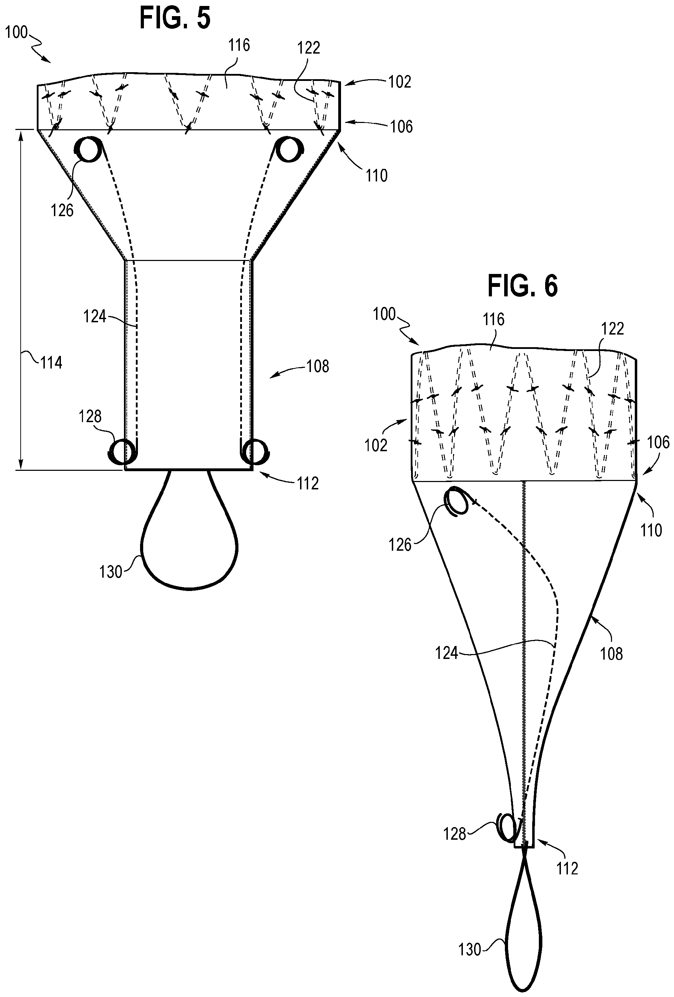

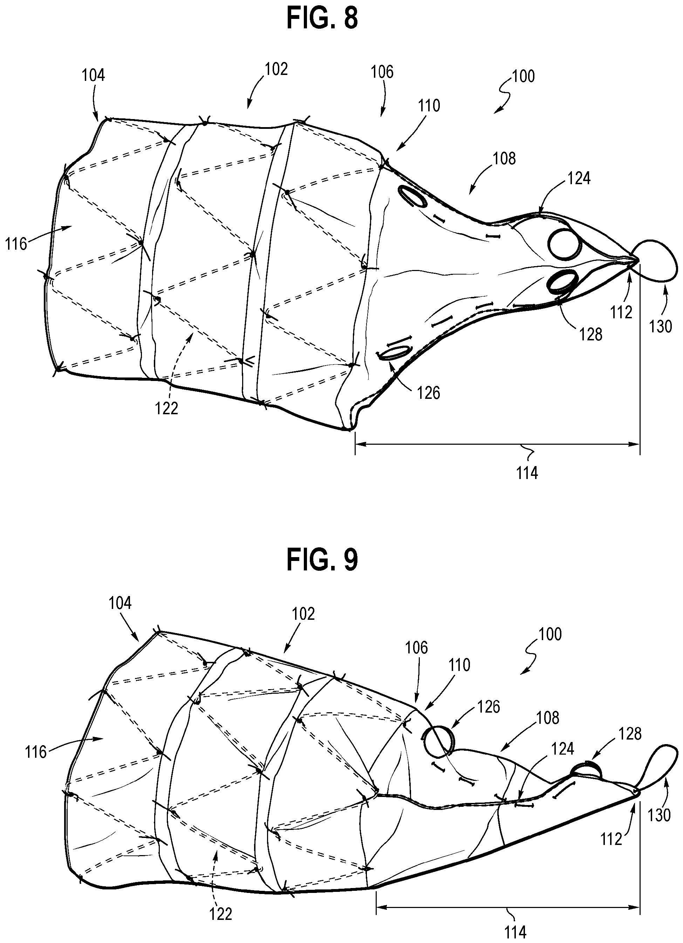

The valve member **108** may be substantially cylindrical or relatively flat. As shown in FIGS. **6** and **9****,** the valve member **108** is mostly flat. This is accomplished by stitching along the sides of the valve member **108** to create this flat configuration. One skilled in the art will appreciate that the valve member **108** may be in various shapes. The desired flat shape of valve member **108** may also be accomplished by cutting the sides of the material forming the valve member **108** and then stitching/sewing those sides together or otherwise reattached to itself (e.g., via a suitable adhesive). As seen in FIGS. **8** and **11****,** the valve member **108** includes two stitch lines.

Referring now to FIGS. **5-6** and **11-12,** the valve member **108** includes the at least one resilient member **124** with a proximal end **126** and a distal end **128.** The at least one resilient member **124** may be at least one elongate resilient member **124** as seen in FIG. **4****.** The at least one resilient member **124** may also be at least one shape-memory material member, such as at least one pre-coiled nitinol wire. Other materials that can be biased to cause the valve member **108** to self-close and adopt the closed and/or deployed condition may also be used, for example, materials with spring characteristics.

As shown in FIGS. **3** and **8****-9,** the at least one resilient member **124** is provided at least partially along the length **114** of valve member **108.** In the depicted embodiment, the at least one resilient member **124** is interwoven longitudinally through the graft material **116** of the valve member **108** at least partially along the length **114** of the valve member **108.** One skilled in the art will appreciate that there are other means of securing or attaching the at least one resilient member **124** to the valve member **108,** for example, by using a suitable adhesive, being incased in one or more pockets, suturing, or sewing. Further, the at least one resilient member **124** terminates distally to the distal end **106** of the support member **102** and forms no part of the support member **102.** Both the proximal end **126** and the distal end **128** of the at least one resilient member **124** are distal to the support member **102.** The at least one resilient member **124** may also terminate proximally to the distal end **112** of the valve member **108,** e.g., the distal end **126** of the at least one resilient member **124** is proximal to the distal end **112** of the valve member **108.** In alternative embodiments, the at least one resilient member **124** may terminate at the distal end **112** of the valve member **108** or extend distally to the distal end **112** of the valve member **108.**

As shown in FIGS. **5-6** and **11-12,** the at least one resilient member **124** is also unattached to the support member **102** and/or the at least one stent **122.** The at least one resilient member **124** and the at least one stent **122** are separate. Further, the proximal end **126** and the distal end **128** of the at least one resilient member **124** are unattached to and/or are free from the valve member **108.** In this embodiment, the proximal end **126** and the distal end **128** of the at least one resilient member **124** are coiled and configured to prevent removal of the at least one resilient member **124** from the valve member **108.** The coiled proximal end **126** and distal end **128** of the at least one resilient member **124** is also advantageous for making the ends **126** and **128** atraumatic and less likely to puncture and/or damage vessel walls. In alternative embodiments, the proximal end **126** and the distal end **128** may not be coiled, or at least one of the proximal end **126** and the distal end **128** may be coiled. Additionally, the proximal end **126** and the distal end **128** may be either external or internal to the valve member **108,** or a combination thereof (e.g., the proximal end **126** is internal while the distal end **128** is external).

In other examples, the resilient member may be configured to prevent removal of the at least one resilient member **124** from the valve member **108** and/or make the proximal end **126** and distal end **128** atraumatic without coiling. For example, the proximal end **126** and distal end **128** may each include a retention structure configured to prevent removal of the at least one elongate resilient member **124** from the valve member **108.** Retention structures may include (but are not limited to) a casing or covering, blunted or rounded tips, ball, knot, wedge, washer, nut, clip, protrusion, other structures that provide a greater width than an opening through which the at least one resilient member **124** is interwoven with the graft material **116,** etc. The proximal end **126** and the distal end **128** may also be embedded into the valve member **108** such as by way of a sleeve or pocket. One skilled in the art will appreciate that there are other means and combinations thereof to prevent removal of the at least one resilient member **124** while also minimizing injury to vessel walls.

As most clearly shown in FIGS. **3** and **8****,** the at least one resilient member **124** is two resilient members **124** located partially along the length **114** of the valve member **108.** The two resilient members **124** are located diametrically opposite of one another. In other words, the two resilient members **124** are on substantially opposite sides of the valve member **108.** Alternative embodiments may include one resilient member **124** along a centerline of the valve member **108** or more than two resilient members **124** spaced equidistant around the valve member **108.** One skilled in the art would appreciate that there are various arrangements with different numbers of resilient members **124** extending partially along the length **114** of the valve member **108.** Further, alternative embodiments may also include at least one resilient member **124** at least partially along a width of the valve member **108** or combinations of resilient members **124** at least partially along both the width and the length **114** of the valve member **108.** Alternative embodiments may also include at least one resilient member **124** attached to flanges at least partially along the length **114** of the valve member **108.** Alternative embodiments may also include at least one resilient member **124** attached to the valve member **108** about its circumference (e.g., the resilient member **124** wraps around the valve member **108).**

The at least one resilient member **124** includes a bias and/or tendency to coil and/or fold. Because the at least one resilient member **124** is attached to the valve member **108** at least partially along its length **114,** the at least one resilient member **124** is configured to fold and/or coil close the valve member **108** and its open distal end **112.** Accordingly, the at least one resilient member **124** also has a tendency to coil and/or fold close the distal end **112** of the valve member **108,** and the valve member **108** is self-closing (or self-shutting, or closes/shuts automatically, etc.). As seen in FIGS. **11-12****,** the at least one resilient member **124** is an elongated configuration. Upon release from a constraining force, the at least one resilient member **124** moves from its elongated configuration as shown in FIGS. **8-10** to a coiled configuration thereby folding and/or coiling close the open distal end **112** of the valve member **108** and moving the device **100** to its deployed condition, as seen in FIGS. **13-15****.** In at least this manner, the at least one resilient member **124** is configured to close the distal end **112** of the valve member **108** in the deployed condition.

The constraining force is provided by an introducer assembly (not depicted). As discussed below, the introducer assembly is a conventional way to deliver the device **100** to a target site within a false lumen. As seen in FIGS. **11-12****,** the valve member **108** includes a loop **130,** which may be a wire, a thread, a suture, or the like. The constraining force is applied by the introducer assembly to the loop **130** to pull and/or move the at least one resilient member **124** into the elongated configuration during delivery of the device **100.** Once the device **100** is in place within the false lumen, the introducer assembly is retracted. During retraction, the introducer assembly releases the constraining force applied to the loop **130,** which allows the at least one resilient member **124** to move from the elongated configuration to the coiled configuration.

To use the implantable medical device **100** of FIGS. **3-15****,** the device **100** is compressed into the introducer assembly in a conventional way. The device **100** is advantageous because the valve member **108** is easily foldable and/or radially compressible and does not significantly limit the compression of the support member **102** as some prior art occluders do.

During introduction, the device **100** is introduced and deployed in a conventional way. Generally, a wire guide is advanced to the treatment site, and a delivery device is then advanced over the wire guide. The delivery device generally comprises an introducer cannula **132** with a distal tip and an outer sheath. The device **100** is carried in a compressed condition between the inner cannula **132** and the outer sheath with the inner cannula **132** passing through the valve member **108.** Typically, a pusher member is included to help advance the device **100** to the desired position in a conventional manner. When the device **100** is at the treatment site within the false lumen, the outer sheath is retracted, allowing the device **100** to expand to the deployed condition and seal against the walls of the false lumen along the length of the support member **102.**

The introducer system is then retracted. The distal end **112** of the valve member **108** is open to allow the inner cannula **132,** the distal tip, and the wire guide to be retracted through the open distal end **112.** During retraction through the open distal end **112,** the constraining force keeping the at least one resilient member **124** in the elongated configuration is released, allowing the at least one resilient member **124** to move to the coiled configuration thereby closing the open distal end **112** of the valve member **108** and moving the device **100** to the deployment condition. The device **100** is advantageous because the valve member **108** self-closes upon retraction of the introducer system while providing improved closure of the valve member **108** to prevent proximal flow in the false lumen.

Once the device **100** is in position, blood in the false lumen will tend to thrombose. This is because blood flowing through the tear will flow against the device **100** in the deployment condition where the valve member **108** and its distal end **112** are folded and/or coiled closed, preventing proximal flow into the valve member **108** and thereby prevent preventing proximal flow in the false lumen. Once the blood in the false lumen has thrombosed, there is no risk for false lumen expansion and risk of rupture.

The subject matter of the present description may also relate, among others, to the following aspects:
In a first aspect, an embodiment of an implantable medical device for preventing backflow into a false lumen of dissection is provided, comprising: an elongate support member including an internal wall forming an internal lumen, a proximal end, and a distal end; an elongate flexible tubular valve member extending distally of the elongate support member and having a length, an open proximal end, and an open distal end, the proximal end of the elongate flexible tubular valve member extending from the distal end of the elongate support member and being configured to be held open thereby in a deployed condition of the implantable medical device, the elongate flexible tubular valve member being unstented along its length, and at least one elongate resilient member extending at least partially along the length of the elongate flexible tubular valve member, the at least one elongate resilient member having a proximal end distal to the elongate support member, a distal end proximal to the open distal end of the elongate flexible tubular valve member, an elongated configuration, and a coiled configuration, wherein the at least one elongate resilient member is unattached to the elongate support member, and wherein the at least one elongate resilient member, upon release from a constraining force, is configured to move from the elongated configuration to the coiled configuration thereby closing the open distal end of the elongate flexible tubular valve member.

In a second aspect, the implantable medical device of the first aspect, wherein the at least one elongate resilient member is interwoven with the elongate flexible tubular valve member at least partially along the length of the elongate flexible tubular valve member.

In a third aspect, the implantable medical device of the second aspect, wherein that each of the proximal end and the distal end of the at least one elongate resilient member includes a retention structure configured to prevent removal of the at least one elongate resilient member from the elongate flexible tubular valve member.

In a fourth aspect, the implantable medical device of the third aspect, wherein the retention structure is a coil.

In a fifth aspect, the implantable medical device of the first aspect, wherein the at least one elongate resilient member is at least one shape-memory material member.

In a sixth aspect, the implantable medical device of the fifth aspect, wherein the at least one shape-memory material member is at least one pre-coiled nitinol wire.

In a seventh aspect, the implantable medical device of the first aspect, wherein the elongate flexible tubular valve member tapers from its proximal end to its distal end along at least part of its length.

In an eighth aspect, the implantable medical device of the first aspect, wherein the at least one elongate resilient member is two elongate resilient members.

In a ninth aspect, the implantable medical device of the eighth aspect, wherein the two elongate resilient members are located diametrically opposite partially along the length of the elongate flexible tubular valve member.

In a tenth aspect, an embodiment of an implantable medical device for preventing backflow into a false lumen of dissection is provided, comprising: an elongate support member including an internal wall forming an internal lumen, a proximal end, and a distal end; a flexible valve member extending distally of the elongate support member, the flexible valve member including a length, an open proximal end, and an open distal end, the proximal end of the flexible valve member being configured to be held open by the elongate support member in a deployed condition of the implantable medical device, and at least one resilient member extending at least partially along the length of the flexible valve member, the at least one resilient member having a proximal end and a distal end that each terminate distally of and are unattached to the elongate support member, wherein the at least one resilient member is configured to close the open distal end of the flexible valve member in the deployed condition of the implantable medical device.

In an eleventh aspect, the implantable medical device of the tenth aspect, wherein the at least one resilient member is interwoven with the flexible valve member at least partially along the length of the flexible valve member.

In a twelfth aspect, the implantable medical device of the eleventh aspect, wherein at least one of the proximal end and the distal end of the at least one resilient member is configured to prevent removal of the at least one resilient member from the flexible valve member.

In a thirteenth aspect, the implantable medical device of the twelfth aspect, wherein at least one of the proximal end and the distal end of the at least one resilient member includes a retention structure configured to prevent removal of the at least one resilient member from the flexible valve member.

In a fourteenth aspect, the implantable medical device of the thirteenth aspect, wherein the retention structure is a coil.

In a fifteenth aspect, the implantable medical device of the tenth aspect, wherein the at least one resilient member is two resilient members, the two resilient members being located diametrically opposite partially along the length of the flexible valve member.

In a sixteenth aspect, the implantable medical device of the tenth aspect, wherein the at least one resilient member is at least one shape-memory material member.

In a seventeenth aspect, the implantable medical device of the sixteenth aspect, wherein the at least one shape-memory material member is at least one pre-coiled nitinol wire.

In an eighteenth aspect, the implantable medical device of the tenth aspect, wherein the at least one resilient member is configured to fold and/or coil close the open distal end of the flexible valve in the deployed condition of the implantable medical device.

In a nineteenth aspect, the implantable medical device of the tenth aspect, wherein the at least one resilient member includes an elongated configuration and a coiled configuration, and wherein, upon release from a constraining force, the at least one resilient member is configured to move from the elongated configuration to the coiled configuration thereby closing the open distal end of the flexible valve member.

In a twentieth aspect, the implantable medical device of the tenth aspect, wherein the elongate support member includes at least one stent, and wherein the at least one resilient member is unattached to the at least one stent.

In a twenty-first aspect, an embodiment of an implantable medical device for preventing backflow into a false lumen of dissection is provided, comprising: a graft material having an internal wall forming an internal lumen, the graft material extending from a proximal end of an elongate support member to an open distal end of a flexible valve member, the flexible valve member extending distally from the elongate support member and having an open proximal end configured to be held open by the elongate support member; at least one stent supporting the internal wall of the elongate support member, and at least one resilient member extending partially along a length of the flexible valve member and being unattached to the at least one stent, wherein the flexible valve member is unstented, and wherein the at least one resilient member includes a tendency to coil and/or fold close the open distal end of the flexible valve member.

In a twenty-second aspect, the implantable medical device of the twenty-first aspect, wherein the at least one resilient member is interwoven with the graft material.

In a twenty-third aspect, the implantable medical device of the twenty-first aspect, wherein the at least one resilient member terminates distally to the elongate support member and proximally to the open distal end of the flexible valve member.

In a twenty-fourth aspect, the implantable medical device of the twenty-third aspect, wherein at least one of a proximal end and a distal end of the at least one resilient member is atraumatic.

In a twenty-fifth aspect, the implantable medical device of the twenty-third aspect, wherein at least one of a proximal end and a distal end of the at least one resilient member includes a retention structure configured to prevent removal of the at least one resilient member from the flexible valve member.

In a twenty-sixth aspect, the implantable medical device of the twenty-fifth aspect, wherein the retention structure is a coil.

In a twenty-seven aspect, the implantable medical device of the twenty-first aspect, wherein the at least one resilient member is at least one shape-memory material member.

In a twenty-eighth aspect, the implantable medical device of the twenty-seventh aspect, wherein the at least one shape-memory material member is at least one pre-coiled nitinol wire.

In a twenty-ninth aspect, the implantable medical device of the twenty-first aspect, wherein the flexible valve member is an elongate flexible tubular valve member.

In a thirtieth aspect, the implantable medical device of the twenty-ninth aspect, wherein the elongate flexible tubular valve member tapers from its proximal end to its open distal end along at least part of its length.

In a thirty-first aspect, the implantable medical device of the thirtieth aspect, wherein the at least one resilient member is two elongate resilient members, and wherein the two elongate resilient members are located diametrically opposite partially along the length of the elongate flexible tubular valve member.

In a thirty-second aspect, the implantable medical device of the twenty-first aspect, wherein the at least one resilient member includes an elongated configuration and a coiled configuration, and wherein, upon release from a constraining force, the at least one resilient member is configured to move from the elongated configuration to the coiled configuration thereby closing the open distal end of the flexible valve member.

All optional and preferred features and modifications of the described embodiments and dependent claims are usable in all aspects of the invention taught herein. Furthermore, the individual features of the dependent claims, as well as all optional and preferred features and modifications of the described embodiments, are combinable and interchangeable with one another.

## Claims

1. An implantable medical device for preventing backflow into a false lumen of dissection, comprising:
an elongate support member including an internal wall forming an internal lumen, a proximal end, and a distal end;
an elongate flexible tubular valve member extending distally of the elongate support member and having a length, an open proximal end, and an open distal end, the proximal end of the elongate flexible tubular valve member extending from the distal end of the elongate support member and being configured to be held open thereby in a deployed condition of the implantable medical device, the elongate flexible tubular valve member being unstented along its length, and
at least one elongate resilient member extending at least partially along the length of the elongate flexible tubular valve member, the at least one elongate resilient member having a proximal end distal to the elongate support member, a distal end proximal to the open distal end of the elongate flexible tubular valve member, an elongated configuration, and a coiled configuration,
wherein the at least one elongate resilient member is unattached to the elongate support member, and
wherein the at least one elongate resilient member, upon release from a constraining force, is configured to move from the elongated configuration to the coiled configuration thereby closing the open distal end of the elongate flexible tubular valve member.

2. The implantable medical device of claim 1, wherein each of the proximal end and the distal end of the at least one elongate resilient member includes a retention structure configured to prevent removal of the at least one elongate resilient member from the elongate flexible tubular valve member.

3. The implantable medical device of claim 2, wherein the retention structure is a coil.

4. The implantable medical device of any preceding claim, wherein the at least one elongate resilient member is at least one of: (a) at least one shape-memory material member; and (b) two elongate resilient members, for example wherein the two elongate resilient members are located diametrically opposite partially along the length of the elongate flexible tubular valve member.

5. An implantable medical device for preventing backflow into a false lumen of dissection, comprising:
an elongate support member including an internal wall forming an internal lumen, a proximal end, and a distal end;
a flexible valve member having a length, an open proximal end, and an open distal end, the proximal end of the flexible valve member extending from the elongate support member and being configured to be held open by the elongate support member in a deployed condition of the implantable medical device, and
at least one resilient member extending at least partially along the length of the flexible valve member, the at least one resilient member having a proximal end and a distal end that each terminate distally of and are unattached to the elongate support member,
wherein the at least one resilient member is configured to close the open distal end of the flexible valve member in the deployed condition of the implantable medical device.

6. The implantable medical device of claim 5, wherein at least one of the proximal end and the distal end of the at least one resilient member is configured to prevent removal of the at least one resilient member from the flexible valve member.

7. The implantable medical device of claim 6, wherein at least one of the proximal end and the distal end of the at least one resilient member includes a retention structure configured to prevent removal of the at least one resilient member from the flexible valve member, for example wherein the retention structure is a coil.

8. The implantable medical device of claim 5, 6 or 7, wherein the at least one resilient member is at least one of:
(a) two resilient members, the two resilient members being located diametrically opposite partially along the length of the flexible valve member; and
(b) at least one shape-memory material member.

9. The implantable medical device of claim any of claims 5 to 8, wherein the at least one resilient member is configured to fold and/or coil close the open distal end of the flexible valve in the deployed condition of the implantable medical device.

10. The implantable medical device of any of claims 5 to 8, wherein the at least one resilient member includes an elongated configuration and a coiled configuration, and wherein, upon release from a constraining force, the at least one resilient member is configured to move from the elongated configuration to the coiled configuration thereby closing the open distal end of the flexible valve member.

11. An implantable medical device for preventing backflow into a false lumen of dissection, comprising:
a graft material having an internal wall forming an internal lumen, the graft material extending from a proximal end of an elongate support member to an open distal end of a flexible valve member, the flexible valve member extending distally from the elongate support member and having an open proximal end configured to be held open by the elongate support member;
at least one stent supporting the internal wall of the elongate support member, and
at least one resilient member extending partially along a length of the flexible valve member and being unattached to the at least one stent,
wherein the flexible valve member is unstented, and
wherein the at least one resilient member includes a tendency to coil and/or fold close the open distal end of the flexible valve member.

12. The implantable medical device of claim 11, wherein the at least one resilient member terminates distally to the elongate support member and proximally to the open distal end of the flexible valve member.

13. The implantable medical device of claim 11 or 12, wherein at least one of a proximal end and a distal end of the at least one resilient member includes a retention structure configured to prevent removal of the at least one resilient member from the flexible valve member, for example wherein the retention structure is a coil.

14. The implantable medical device of any of claims 11 to 13, wherein the at least one resilient member is two elongate resilient members, and wherein the two elongate resilient members are located diametrically opposite partially along the length of the elongate flexible tubular valve member.

15. The implantable medical device of claim 14, wherein the at least one resilient member includes an elongated configuration and a coiled configuration, and wherein, upon release from a constraining force, the at least one resilient member is configured to move from the elongated configuration to the coiled configuration thereby closing the open distal end of the flexible valve member.
